Europäisches Patentamt

(19) European Patent Office

Office européen des brevets

(11) EP 0 647 118 B1

(12) EUROPEAN PATENT SPECIFICATION

(45) Date of publication and mention
of the grant of the patent:
**19.03.1997 Bulletin 1997/12**

(21) Application number: **93918132.7**

(22) Date of filing: **18.06.1993**

(51) Int. Cl.$^6$: **A61B 5/0215**, G01L 1/24

(86) International application number:
**PCT/US93/06105**

(87) International publication number:
**WO 94/00051 (06.01.1994 Gazette 1994/02)**

(54) **BLOOD PRESSURE TRANSDUCER**

BLUTDRUCK-WANDLER

TRANSDUCTEUR DE PRESSION ARTERIELLE

(84) Designated Contracting States:
**DE FR GB IT**

(30) Priority: **29.06.1992 JP 170949/92**
**01.06.1993 JP 130516/93**

(43) Date of publication of application:
**12.04.1995 Bulletin 1995/15**

(73) Proprietor: **MINNESOTA MINING AND MANUFACTURING COMPANY**
**St. Paul, Minnesota 55133-3427 (US)**

(72) Inventor: **TAKAKI, Shunsuke**
**Sagamihara-city, Kanagawa-pref. 229 (JP)**

(74) Representative: **Hilleringmann, Jochen, Dipl.-Ing. et al**
**Patentanwälte**
**von Kreisler-Selting-Werner,**
**Bahnhofsvorplatz 1 (Deichmannhaus)**
**50667 Köln (DE)**

(56) References cited:
**EP-A- 0 392 897          GB-A- 1 001 809**
**US-A- 4 711 246          US-A- 5 089 697**

• **RADIOLOGY vol. 156, no. 2 , August 1985 page 548 C.F.ROOS ET AL. 'Fiber-optic Pressure Transducer for Use Near MR Fields'**

## Description

### FIELD OF THE INVENTION

This invention relates to a blood pressure transducer in medical measuring instruments.

### BACKGROUND OF THE INVENTION

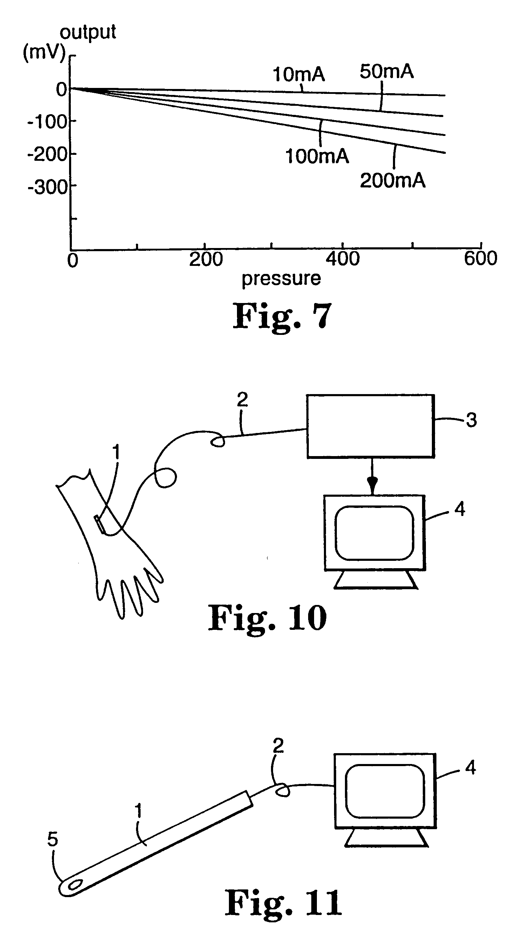

Invasive direct blood pressure measurement is often performed on patients in the intensive care unit or the coronary care unit of a hospital, during heart surgery, and so forth. This method can observe the blood pressure waveform with a higher level of accuracy than a non-invasive indirect blood pressure transducer, and can also measure a local pressure such as an internal pressure of the heart. The following three systems have been employed for this purpose and are examples of invasive direct blood pressure transducers. Figs. 10 and 11, discussed below, are explanatory views of conventional systems. Hereinafter, these three systems and their drawbacks will be explained.

(1) External Sensor System Using Semiconductor Pressure Transducer:

This first system is illustrated in Fig. 10 and is the most widely used type at present. The transducers of this type have been put on the market by a large number of manufacturers. A catheter 1 filled with a physiological saline solution through a pressure tube 2 is inserted into a blood vessel of the arm of a patient, and the other end of the pressure tube 2 is connected to a semiconductor pressure transducer 3. This transducer 3 detects the change of the pressure of the saline solution, and a monitor 4 observes the change of the blood pressure.

This system is convenient and can accurately measure a patient's blood pressure for an extended period of time. Since many of the transducers are of a disposable type, there is a less danger of infection to others. Nonetheless, since the semiconductor pressure transducer 3 is of an electrical type, there is the danger of electric shock to the patient due to the failure of the apparatus, and the like. Furthermore, since the semiconductor pressure transducers 3 are complicated in structure, the problem of the high production cost is left unsolved when using them as a disposable device.

(2) Sensor System Using Catheter with Semiconductor Pressure Transducer at its tip:

This second system is shown in Fig. 11. A small semiconductor pressure transducer 5 is assembled into the tip of a catheter 1, and the pressure of a local portion (e.g. internal chamber of the heart, large blood vessels, etc) is observed by a monitor unit 4 by inserting the catheter 1 into the blood vessel or chamber. The physiological saline solution is charged into a pressure tube 2 in the same way as in the first system. This system, too, has already been put on the market.

According to this system, however, it is difficult to produce a probe having as small a diameter as the catheter 1. Furthermore, this system is not suitable for taking measurements over a prolonged time, e.g., by leaving the probe inside a small blood vessel. Finally, the danger of electric shock to the patient is even greater than in the first system described above.

(3) Sensor System Using Catheter with Optical Fiber:

This third system has the same basic structure as the second system. However, to eliminate the potential problem of electric shock inherent in the second system, this system uses a sensor comprising the combination of an optical fiber and a diaphragm, etc, in place of the semiconductor pressure sensor 5 (see KOKAI Japanese Unexamined Patent Publication No. 62-47335).

While the problem of harmful electric shock can be avoided by using this optical system, other problems remain. For example, additional components such as a diaphragm, etc., become necessary, therefore, the sensor becomes larger. This system is also unsuitable for taking measurements over a prolonged time.

Furthermore, US-A-5 089 697 discloses an optical pressure transducer, wherein two ends of fibres, facing each other, move away from each other upon applying pressure. Roos et al. (in: Radiology, vol. 156, page 548; 1985) use a moving shutter mounted on a membrane.

### SUMMARY OF THE INVENTION

The present invention provides a blood pressure transducer. The blood pressure transducer according to the present invention uses an optical fiber made of a transparent elastomer as the pressure transducer, and provides an economical, disposable structure which is safe due to its reduced potential to cause an undesirable electric shock. The transducers of the present invention are capable of continuously measuring arterial and venous pressures over a prolonged period of time and can easily be calibrated.

### BRIEF DESCRIPTION OF THE DRAWINGS

FIG. 1 is a structural view of principal portions of an invasive direct blood pressure transducer according to the present invention.

FIG. 2 is an explanatory view useful for explaining the operation principle of the present invention.

FIG. 3 is a detailed structural view of a pressure transducer according to the present invention.

FIG. 4 is an overall structural view of the invasive direct blood pressure transducer according to the present invention.

FIG. 5 shows response examples of the blood pressure transducer of the present invention.

FIG. 6 is a graph showing the effect of the present invention.

FIG. 7 is a graph showing the effect of the present invention.

FIG. 8 is a structural view of an invasive direct blood pressure transducer according to another embodiment of the present invention.

FIG. 9 is a detailed structural view of the light measuring unit shown in Fig. 8.

FIG. 10 is an explanatory view of a conventional system.

FIG. 11 is an explanatory view of a conventional system.

## DETAILED DESCRIPTION OF THE INVENTION

In view of the problems described above in the background section, the present invention provides a blood pressure transducer which is capable of continuously measuring the arterial and venous pressures by means of a pressure transducer through a catheter, and which eliminates the danger of electric shock. The transducer is small in size, can be calibrated easily, and is economical and disposable. The transducer comprises an optical fiber made of a transparent elastomer as a pressure transducer.

The present invention provides an invasive direct blood pressure transducer of an external sensor type comprising a pressure tube connected to one of the ends of a catheter and a pressure transducer connected to the other end of the pressure tube. A part of the pressure transducer is composed of an optical fiber made of a transparent elastomer, and wherein the pressure transducer is equipped with a light source at one of the ends thereof and with an optical sensor at the other end.

In a preferred embodiment, the pressure transducer is quipped with a light source at one of the ends thereof and with an optical sensor at the other end thereof, the pressure tube is connected to the optical fiber at an intermediate part of the optical fiber, light is allowed to be incident from the light source, and, as shown by dotted lines in Fig. 2, the optical sensor detects the change of a light intensity of the incident light resulting from the change of a transmission factor of the optical fiber in response to the change of an internal pressure of a blood vessel.

Further, in another embodiment, the pressure transducer is quipped with a sensitivity adjustment screw for adjusting variance of sensitivity of said optical fiber.

Still further, in another embodiment, the pressure transducer is quipped with a light measuring unit including a light source, an optical sensor and a light splitter at one of the ends thereof and with a reflection unit at the other end thereof for reflecting a light from the light source, the pressure tube is connected to the optical fiber at an intermediate part of the optical fiber, the light is allowed to be incident from the light source through the light splitter, and the optical sensor detects the change of a light intensity of a reflection light reflected by the reflection unit resulting from the change of a transmission factor of the optical fiber in response to the change of an internal pressure of a blood vessel.

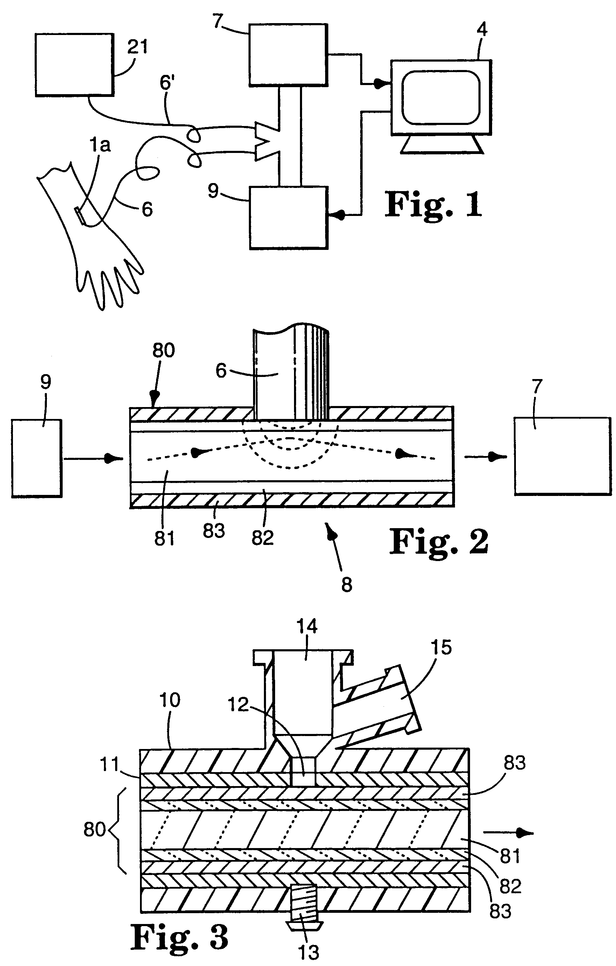

As described above, the present invention provides two systems for detecting the change of an amount of light caused by the change of the blood pressure, i.e., one system utilizing a transmission light as shown in Fig. 1, and the other system utilizing a reflected light as shown in Fig. 8.

FIG. 1 is a structural view of the principal portions of an invasive direct blood pressure transducer according to the present invention. As shown in the drawing, the invasive direct blood pressure transducer according to the present invention comprises a pressure transducer 8, a light source 9, an optical sensor 7, a monitor unit 4, a tube 6 and 6' (optionally comprising a vinyl polymer), and a catheter 1a. The transducer according to the present invention uses an optical blood pressure transducer 8 comprising an optical fiber made of a transparent elastomer.

A liquid such as a physiological saline solution is packed into the pressure receiving portion of the pressure transducer 8, the tube 6 and the catheter 1a. The pressure of this solution changes with the change of the pressure of a blood vessel, so that the internal pressure of the blood vessel can be transmitted to the pressure transducer 8.

FIG. 2 is an explanatory view useful for explaining the principle of operation of the present invention. The optical fiber 80, made of a transparent elastomer, used for the pressure transducer of the present invention exhibits the following operation. The optical fiber 80 comprises a core portion 81 made of a transparent elastomer having a high refractive index, a cladding portion 82 made of a transparent elastomer having a low refractive index and a jacket portion 83 made of an opaque (e.g., black) elastomer which serves to prevent the introduction of any external light into the sensor. When light is allowed to be incident into one of the ends of the optical fiber 80 from the light source 9, the light undergoes total reflection on the boundary between the core 81 and the cladding 82 and exits the other end of the optical fiber 80.

When the internal pressure of the blood vessel acts on part of the side surface of this optical fiber 80 through the physiological saline solution packed into the tube 6, the inside of the optical fiber 80 receiving this pressure undergoes deformation as represented by dotted line, and the boundary between the core 81 and the cladding 82 is disturbed, so that the intensity of the outgoing light drops. On the other hand, since the optical fiber 80 is made of an elastomer it returns to its original state unless the pressure is maintained thereto, and the light intensity returns to the original level, as well. The change of the light intensity in this instance is detected by the optical sensor 7 receiving the outgoing light, and the monitor unit 4 observes this change so as to measure the blood pressure.

Suitable elastomers for use in the present invention as the transparent elastomer for the optical fiber 80 include: silicone elastomers, acrylate elastomers, fluorocarbon elastomers, vinyl chloride elastomers, ethylene propylene elastomers, etc. After the core portion is molded from these materials by, for example, a known extrusion molding method or casting method, the cladding portion and the jacket portion are molded by a coating method.

Known light emitting diodes, laser, xenon lamps, halogen lamps, etc, can be used as the light source 9. known devices such as photo-diodes, photo-multipliers, etc, can be used as the optical sensor 7.

FIG. 3 is a detailed structural view of the transducer portion. The optical fiber 80 is disposed in a cell 10 and is held in place by a packing material 11 which fixes the optical fiber to the cell at sites other than the site of a pressure sensitive hole 12. A sensitivity adjustment screw 13 is fitted to the cell immediately below, and opposite to, the pressure sensitive hole 12. When this screw 13 is turned up and down, a pressure can be applied to the core 81 and the cladding 82 through the jacket portion 83, and the sensitivity can be adjusted by intentionally causing deformation of the optical fiber 80 by this externally applied pressure. In other words, variance of the sensitivity of a plurality of pressure transducers can be calibrated to a predetermined sensitivity by turning up and down this screw 13. Reference numeral 14 denotes a flange portion for connecting the tube 6 and reference numeral 15 denotes the flange portion for connecting the vinyl tube 6', which connects the flash bag 21, through a valve 16.

Suitable materials for the cell 10 include polymeric materials such as polycarbonate, acrylate resin, epoxy resin, polyethylene, polypropylene, polystyrene, fluororesin, etc. Alternately, one may use a suitable metal or ceramic material to form the cell. If desired, one may optionally employ an opaque material (i.e., opaque to the wavelength of light being transmitted from the light source) for the cell. In such a case, the opaque elastomeric jacket 83 may be omitted. In general, however, transparent materials are preferred for use as the cell so that the operator may check visually for occurrence of air bubbles in the vicinity of the pressure sensitive hole 12. Suitable packing materials 11 for fixing the optical fiber include materials such as silicone resin, acrylate resin, fluororesin, urethane resin, vinyl chloride, ethylene-propylene resin, etc. A plastic screw or metallic screw can be used for the sensitivity adjustment screw 13.

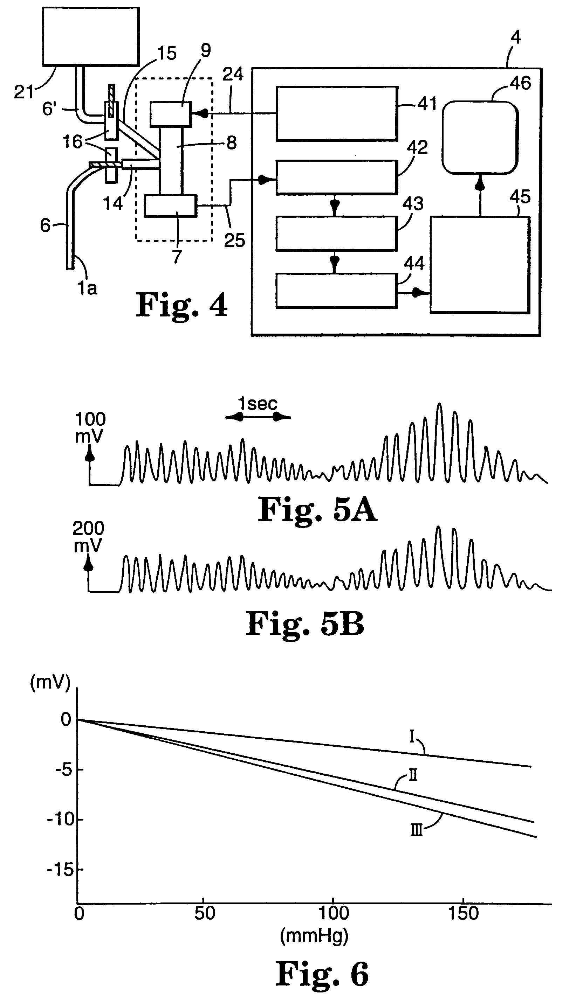

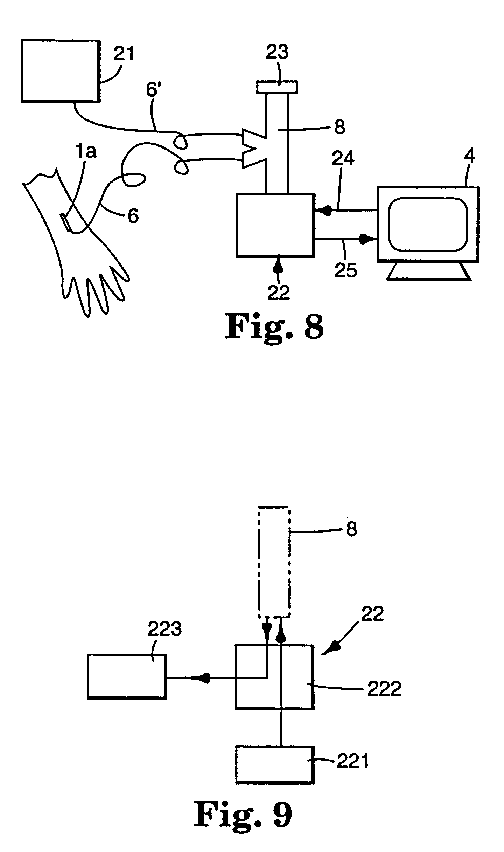

FIG. 4 is an overall structural view of the invasive direct blood pressure transducer according to the present invention. To calibrate the invasive direct blood pressure transducer of the present invention, sensitivity adjustment of the pressure transducer 8 and sensitivity adjustment of the monitor unit 4 are necessary. The sensitivity of the pressure transducers 8 immediately after their production is mutually different due to a fine difference of structure inside the cell 10. Therefore, the sensitivity is adjusted to substantially an equal level by means of the sensitivity adjustment screw 13 of each pressure transducer 8. The pressure transducers 8 are preserved after sterilization, and are fitted to the monitor unit 4 when they are used. At this time, the intensity of light transmission through the optical fiber 80 is generally different from that of the initial calibration depending on factors such as the fitting positions of the light source 9 and the optical sensor 7. To compensate for these differences, the intensity of the light source 9 may be adjusted so that the intensity of the transmitted light becomes standardized at an arbitrary pressure (generally, at 0 mmHg). To standardize the base line of calibration, an auto-zero circuit which compulsorily makes the output signal zero is operated when the transducer is maintained at a pressure of 0 mmHg. Next, the light intensity of the light source 9 is adjusted in such a way that the intensity of the transmission light is standardized at a known valve when the transducer is maintained at a pressure of 200 mmHg. In this way, each pressure transducer can be calibrated easily.

In the monitor unit 4, reference numeral 41 denotes a stabilization unit for stabilizing light power of the light source 9; 42 is an amplifier for converting the light intensity detected by the optical sensor to a voltage and amplifying the voltage; 43 is a filter for picking up only those frequencies necessary for the measurement; 44 is a convertor for converting the voltage obtained by the filter 43 to a digital value; 45 is a microprocessor for converting the digital voltage value obtained by the convertor 44 to an actual blood pressure value; and 46 is a display unit for displaying the result of the microprocessor 45.

An example of each constituent component will now be explained in further detail. The following examples are offered to aid in the understanding of the present invention and are not to be construed as limiting the scope thereof.

## EXAMPLES

### Example 1

#### Fabrication of an optical fiber

A transparent silicone elastomer of a two-liquid RTV type (Sylgard™ 184, available from Dow Corning, Midland, MI) and having a refractive index of 1.430 was sufficiently mixed and then defoamed. The elastomer was charged into a vinyl chloride tube having an inner diameter of 1 mm and was cured at 65°C for 4 hours. Thereafter, the tube was put into tetrahydrofuran and dissolved to provide a core of the silicone elastomer. After being washed and dried, the resulting core was dipped into a sufficiently mixed and defoamed silicone elastomer of a two-liquid RTV type (SE1740, available from Toray-Daw) which has a refractive index of 1.403. After a cladding was thus dip-coated, the resulting assembly was cured. There was thus obtained an optical fiber having a diameter of about 1 mm and a numerical aperture ("NA") of 0.28. "Numerical aperture" as used herein is given by the following formula:

$$NA = \sqrt{n_{core}^2 - n_{clad}^2}$$

where, $n_{core}$ is the refractive index of a core portion, and $n_{clad}$ is the refractive index of a clad portion. The optical fiber was then dipped into a silicone elastomer of a black two-liquid RTV type (SILPOT™ 170, available from Dow Corning), and was cured after dip-coating. In this way, an optical fiber for a pressure transducer could be fabricated.

## Example 2

### Fabrication of an optical pressure transducer

In the structure shown in Fig. 3, the cell 10 was made of polycarbonate, and had a through hole for the optical fiber 80, a pressure sensitive hole 12, a flange for connecting the pressure tube 14, a flange for connecting the flash tube 15, etc. The optical fiber of Example 1 was passed through the through hole, and the sensitivity adjustment screw and the pressure sensitive hole blind screw were fitted. A silicone resin containing an inorganic material (Imprint™ available from 3M Co., St. Paul, MN) was packed and cured between the optical fiber and the cell. After curing, the optical fiber 80 and the silicone resin swelling out from the cell 10 were cut off, and the blind screw was removed thereby defining a pressure sensitive hole. In this way, the disposable pressure transducer could be fabricated easily.

## Example 3

### Blood pressure transducer

In Figs. 3 and 4, a blood pressure transducer comprising a sensor head, a monitor unit, a catheter, a flash bag and a vinyl tube is shown. The outside of the sensor head was made of a metal thereby providing electromagnetic shielding, and its top could be opened and closed. A light source unit 9 consisting of a light emitting diode and a monitor sensor, a pressure transducer 8, an optical sensor unit 7 and a connector constituted the sensor head. After the optical sensor unit was inserted into the light source unit, the pressure transducer was then inserted. The vinyl tube 6 was fitted to the pressure tube connection flange 14, and a three-way valve 16 and a flash bag unit were fitted to the flash flange 15.

In this case, the flash bag unit is a pressurization type, and it applies pressure to the physiological saline (to which a heparine, i.e., an anti-clot agent, is added), and supplies the pressurized saline to the pressure transducer 8 through the vinyl tube 6' so that it is possible to prevent the coagulation of the blood.

Further, although detailed explanation will be given in Figs. 8 and 9, a pressure transducer using reflected light comprises a reflection plate fitted to the end of the pressure transducer 8, and a light measuring unit connected to the other end of the pressure transducer. Further, the pressure tube is connected to the optical fiber at an intermediate part thereof.

Figs. 5(A) and 5(B) show examples of responding waveforms when the blood pressure transducer of the present invention was fitted to a calibration pressure generator. The waveform (A) represents the response of the blood pressure transducer and the waveform (B) represents the response of the pressure generator. As can be understood clearly by comparing the waveforms (A) and (B) with each other, there is obtained substantially the same waveform. The pressure sensitivity was about 1.0 mV/mmHg. This sensitivity could be changed by means of the sensitivity adjustment screw of the transducer and by varying the light intensity.

Figs. 6 and 7 are graphs useful for explaining the effect of the present invention. Fig. 6 illustrates the effect of the sensitivity adjustment screw and Fig. 7 illustrates the effect of varying the light emission intensity of the LED light source. In Fig. 6, the ordinate represents the sensitivity (mV) of the optical sensor and the abscissa represents the pressure of the solution inside the vinyl tube. Straight lines I, II and III represent the cases where the sensitivity adjustment screw was turned 3/4, 1/2 and 1/4 revolutions, respectively. The sensitivity of the optical fiber can thereby be adjusted and variance from other sensors could be eliminated by turning the sensitivity adjustment screw in this way. The ordinate in Fig. 7 represents the sensitivity of the optical sensor and the abscissa represents the pressure of the solution inside the vinyl tube. The graph represents the relation between the output voltage and the pressure when the current caused to flow through the LED was changed to 10, 50, 100 and 200 (mA), respectively. In this way, the sensitivity could be adjusted by changing the LED current. This blood pressure transducer exhibited a linear relationship over a range of more than 300 mmHg, and the response to the heart rate was at least 500 times/min.

Figs. 8 and 9 are structural views of an invasive direct blood pressure transducer according to another embodiment of the present invention. In Fig. 8, reference number 21 denotes a flash bag, 22 a light measuring unit, and 23 a light reflection plate. The light measuring unit 22 is formed by a light source 221, a light splitter 222 and an optical sensor 223 as shown in Fig. 9. In this structure, the light from the light source 221 of the light measuring unit 22 is input to the optical fiber 80 through the light splitter (i.e., beam splitter), and sent into the optical fiber 80 to which the vinyl tube 6 is connected. All light passing through the optical fiber 80 is reflected by the light reflection plate 23 which is fitted to the other end of the optical fiber 80.

The reflection light from the light reflection plate 23 passes through the optical fiber 80 again, changes the light path at a right angle to an incident path by using a half-mirror of the light splitter 23 so as to split a part of the reflection light, and the reflection light is detected by the optical sensor 223 in the light measuring unit 22. Accordingly, the optical sensor 223 detects the intensity of the reflection light. Further, this intensity of the reflection light is converted to the blood pressure to display it on the monitor 4.

In this embodiment, as explained above, the light splitter 222 is provided for sending the reflection light to the optical sensor 223 by using the half mirror. As another example, the beam splitter, a light directional coupler, etc., are usable for the light splitter 222.

In the optical fiber 8, first, the intensity of the incident light from the light source 9 is changed in response to the change of the blood pressure at the pressure sensitive hole 12. Next, the attenuated light is reflected by the reflection plate 23 and is changed again in response to the change of the blood pressure at the same portion 12. Accordingly, since the intensity of the light is twice changed within the optical fiber 80 it is possible to obtain a larger change of the intensity of the light than obtained in the first embodiment in which only the incident light is utilized.

Still further, as explained above, the reflection plate 23 is provided for reflecting the light from the light source 221 through the optical fiber 80. Suitable reflection plates include: a metal plate, a plastic plate, a glass plate, a metal evaporated film, etc.

As explained above, since the light source, the optical sensor and the light splitter are integrated within the light measuring unit, it is possible to achieve the simplified structure.

**Claims**

1. A blood pressure transducer system, comprising:

   - a catheter (1a),
   - a pressure tube (6) connected to one of the ends of said catheter (1a) wherein said pressure tube (6) and said catheter (1a) are in fluid communication, and
   - a pressure transducer (8) connected to the other end of said pressure tube (6), wherein said pressure transducer (8) comprises an optical fiber (80) made of a transparent elastomer and having a deformable side surface, wherein said pressure transducer (8) comprises a light source (9;221) and an optical sensor (7;223), wherein said pressure tube (6) is connected to said optical fiber (80) at an intermediate part of said optical fiber (80), and wherein said optical sensor (7;223) is capable of detecting the change of the intensity of the light of said light source (9;221) in response to the change of pressure exerted through said pressure tube (6) on the deformable side surface of said optical fiber (80).

2. A pressure transducer system according to claim 1, characterized in that said light source (9) is arranged to introduce the light at the one end of said optical fiber (80) and that said optical sensor (7) is arranged to receive the light transmitted through said optical fiber (80) and output from the other end of said optical fiber (80).

3. A pressure transducer system according to claim 1, characterized in that said light source (221) and said optical sensor (223) both are arranged so as to introduce light in said optical fiber (80) into and, respectively, to receive the light transmitted through said optical fiber (80) from the same end of said optical fiber (80), and that said pressure transducer (8) further comprises a reflector (23) at the other end of said optical fiber (80)

4. A pressure transducer system according to claim 3, characterized in that said pressure transducer (8) is equipped with a light measuring unit (22) including a light source (221), an optical sensor (223) and a light splitter (222) at one of the ends thereof and with a reflection means (23) at the other end thereof for reflecting a light from said light source (221), said pressure tube (6) is connected to said optical fiber (80) at an intermediate part of said optical fiber (80), said light is allowed to be incident from said light source (221) through said light splitter (222), and said optical sensor (223) detects the change of a light intensity of a reflection light reflected by said reflection means (23) resulting from the change of a transmission factor of said optical fiber (80) in response to the change of pressure exerted through said pressure tube (6) on the deformable side surface of said optical fiber (80).

5. A pressure transducer system according to claim 1, characterized in that said optical fiber (80) comprises a core portion (81) made of a transparent elastomer having a high refractive index, and a cladding portion (82) made of a transparent elastomer having a low refractive index.

6. A pressure transducer system according to claim 1, characterized in that said optical fiber (80) is disposed in a cell (10) and is held in place by means of a packing material (11) which fixes the optical fiber (80) to the cell (10), and that said cell (10) comprises a flange portion (14) for connecting said pressure tube (6) thereto.

7. A pressure transducer system according to claim 6, characterized in that said cell (10) further comprises a sensitivity adjustment screw (13) for adjusting the variance of sensitivity of said optical fiber (80).

8. A pressure transducer system according to claim 5, characterized in that said optical fiber (80) further comprises a jacket portion (83) made of an opaque elastomer.

9.  A pressure transducer system according to claim 1, characterized in that said transparent elastomer is selected from the group consisting of silicone elastomers, acrylate elastomers, fluorocarbon elastomers, vinyl chloride elastomers, and ethylene propylene elastomers.

10. A pressure transducer system according to claim 5, characterized in that the refractive indices of said high refractive index elastomer and said low refractive index elastomer have a difference of at least 0.02.

11. A pressure transducer, comprising:

    -   an optical fiber (80) made of a transparent elastomer and having a deformable side surface,
    -   a light source (9;221) and
    -   an optical sensor (7;223),
    -   wherein said optical fiber (80) is adapted to receive a pressure tube (6) at an intermediate part of said optical fiber (80), and
    -   wherein said optical sensor (7;223) is capable of detecting the change of the intensity of the light of said light source (9;221) in response to the change of pressure exerted through said pressure tube (6) on the deformable side surface of said optical fiber (80).

12. A pressure transducer according to claim 11, characterized in that said light source (9) is arranged to introduce the light at the one end of said optical fiber (80) and that said optical sensor (7) is arranged to receive the light transmitted through said optical fiber (80) and output from the other end of said optical fiber (80).

13. A pressure transducer according to claim 11, characterized in that said light source (221) and said optical sensor (223) both are arranged so as to introduce light in said optical fiber (80) into and, respectively, to receive the light transmitted through said optical fiber (80) from the same end of said optical fiber (80), and that said pressure transducer (8) further comprises a reflector (23) at the other end of said optical fiber (80).

14. A pressure transducer according to claim 13, characterized in that there is provided a light measuring unit (22) including a light source (221), an optical sensor (223) and a light splitter (222) at one of the ends thereof and with a reflection means (23) at the other end thereof for reflecting a light from said light source (221), said pressure tube (6) is connected to said optical fiber (80) at an intermediate part of said optical fiber (80), said light is allowed to be incident from said light source (221) through said light splitter (222), and said optical sensor (223) detects the change of a light intensity of a reflection light reflected by said reflection means (23) resulting from the change of a transmission factor of said optical fiber (80) in response to the change of pressure exerted through said pressure tube (6) on the deformable side surface of said optical fiber (80).

15. A pressure transducer according to claim 11, characterized in that said optical fiber (80) comprises a core portion (81) made of a transparent elastomer having a high refractive index, and a cladding portion (82) made of a transparent elastomer having a low refractive index.

16. A pressure transducer according to claim 11, characterized in that said optical fiber (80) is disposed in a cell (10) and is held in place by means of a packing material (11) which fixes the optical fiber (80) to the cell (10), and that said cell (10) comprises a flange portion (14) for connecting said pressure tube (6) thereto.

17. A pressure transducer according to claim 16, characterized in that said cell (10) further comprises a sensitivity adjustment screw (13) for adjusting the variance of sensitivity of said optical fiber (80).

18. A pressure transducer according to claim 15, characterized in that said optical fiber (80) further comprises a jacket portion (83) made of an opaque elastomer.

19. A pressure transducer according to claim 11, characterized in that said transparent elastomer is selected from the group consisting of silicone elastomers, acrylate elastomers, fluorocarbon elastomers, vinyl chloride elastomers, and ethylene propylene elastomers.

20. A pressure transducer according to claim 15, characterized in that the refractive indices of said high refractive index elastomer and said low refractive index elastomer have a difference of at least 0.02.

21. A method of measuring the pressure of a fluid, comprising the steps of:

    -   transmitting light through an elastomeric optical fiber (80),
    -   deforming part of the side surface of said elastomeric optical fiber (80) by imposing a pressurized fluid on said surface, and
    -   detecting the intensity of the light transmitted through the optical fiber (80) by means of an optical sensor (7;223), so as to measure the fluid pressure.

22. A method according to claim 21, characterized by the further steps of:

- measuring the intensity of said transmitted light when the side surface of said optical fiber (80) has imposed thereon a first known pressure,
- measuring the intensity of said transmitted light when the side surface of said fiber (80) has imposed thereon a second known pressure;
- calculating the relationship between said measured intensities and said first and second known pressures, and
- calculating the pressure of a fluid by using said relationship to convert the intensity of transmitted light when the side surface of said optical fiber (80) has imposed thereon the pressure of said fluid.

23. A method according to claim 22, characterized in that said calculation steps are performed using a microprocessor (45).

**Patentansprüche**

1. Blutdruckwandlersystem mit:

   - einem Katheter (1a),

   - einem mit einem Ende des Katheters (1a) verbundenen Druckschlauch (6), wobei der Druckschlauch (6) und der Katheter (1a) in Fluidverbindung stehen, und

   - einem mit dem anderen Ende des Druckschlauchs (6) verbundenen Druckwandler (8), wobei der Druckwandler (8) eine optische Faser (80) aus einem transparenten Elastomer mit einer verformbaren Seitenfläche aufweist, wobei der Druckwandler (8) eine Lichtquelle (9; 221) und einen optischen Sensor (7; 223) aufweist, wobei der Druckschlauch (6) mit der optischen Faser (80) in einem Mittelbereich der optischen Faser (80) verbunden ist, und wobei der optische Sensor (7; 223) in der Lage ist, die Veränderung der Lichtintensität der Lichtquelle (9; 221) zu erkennen, die in Reaktion auf eine Veränderung des Drucks bewirkt wird, der über den Druckschlauch (6) auf die verformbare Seitenfläche der optischen Faser (80) ausgeübt wird.

2. Druckwandlersystem nach Anspruch 1, dadurch gekennzeichnet, daß die Lichtquelle (9) derart angeordnet ist, daß sie Licht am einen Ende der optischen Faser (80) einleitet, und daß der optische Sensor (7) derart angeordnet ist, daß er das durch die optische Faser (80) transmittierte und am anderen Ende der optischen Faser (80) ausgegebene Licht empfängt.

3. Druckwandlersystem nach Anspruch 1, dadurch gekennzeichnet, daß die Lichtquelle (221) und der optische Sensor (223) beide derart angeordnet sind, daß sie an dem selben Ende der optischen Faser (80) Licht in die optische Faser (80) leiten bzw. das durch die optische Faser geleitete Licht empfangen, und daß der Druckwandler (8) ferner einen Reflektor (23) am anderen Ende der optischen Faser (80) aufweist.

4. Druckwandlersystem nach Anspruch 3, dadurch gekennzeichnet, daß der Druckwandler (8) mit einer Lichtmeßeihheit (22), die eine Lichtquelle (221), einen optischen Sensor (223) und einen Lichtteiler (222) an einem ihrer Enden aufweist, und an ihrem anderen Ende mit einer Reflektionseinrichtung (23) zum Reflektieren des von der Lichtquelle (221) kommenden Lichts versehen ist, daß der Druckschlauch (6) mit der optischen Faser (80) an einem Mittelbereich der optischen Faser (80) verbunden ist, daß das Licht von der Lichtquelle (221) durch den Lichtteiler (222) einfallt, und daß der optische Sensor (223) die Veränderung der Lichtintensität von Reflektionslicht, das von der Reflektionseinrichtung (23) reflektiert wird, erkennt, die aus der Veränderung eines Transmissionsfaktors der optischen Faser (80) resultiert, die sich in Reaktion auf die Veränderung des durch den Druckschlauch (6) auf die verformbare Seitenfläche der optischen Faser (80) aufgebrachten Drucks ergibt.

5. Druckwandlersystem nach Anspruch 1, dadurch gekennzeichnet, daß die optische Faser (80) einen Kern (81) aus einem transparenten Elastomer mit einem hohen Refraktionsindex und einem Mantel (82) aus einem transparenten Elastomer mit einem niedrigen Refraktionsindex aufweist.

6. Druckwandlersystem nach Anspruch 1, dadurch gekennzeichnet, daß die optische Faser (80) in einer Zelle (10) angeordnet ist und durch ein Füllmaterial (11) gehalten ist, das die optische Faser (80) in der Zelle (10) fixiert, und daß die Zelle (10) einen Flanschbereich (14) zum Verbinden des Druckschlauchs (6) mit dieser aufweist.

7. Druckwandlersystem nach Anspruch 6, dadurch gekennzeichnet, daß die Zelle (10) ferner eine Empfindlichkeiteinstellschraube (13) zum Einstellen des Empfindlichkeitsbereichs der optischen Faser (80) aufweist.

8. Druckwandlersystem nach Anspruch 5, dadurch gekennzeichnet, daß die optische Faser (80) ferner eine Hüllen (83) aus einem opaken Elastomer aufweist.

9. Druckwandlersystem nach Anspruch 1, dadurch gekennzeichnet, daß das transparente Elastomer aus der Gruppe gewählt ist, die aus Silikonelastomeren, Acrylatelastomeren, Fluorkarbonelastomeren, Vinylchloridelastomeren und Ethylen-Propylenelastomeren besteht.

10. Druckwandlersystem nach Anspruch 5, dadurch gekennzeichnet, daß die Refraktionsindizes des Elastomers mit dem hohen Refraktionsindex und des Elastomers mit dem niedrigen Refraktionsindex einen Unterschied von wenigstens 0,02 aufweisen.

11. Druckwandler mit:

- einer optischen Faser (80) aus einem transparenten Elastomer und mit einer verformbaren Seitenfläche,

- einer Lichtquelle (9; 221) und

- einem optischen Sensor (7; 223),

- wobei die optische Faser (80) in der Lage ist, an einem Mittelbereich der optischen Faser (80) einen Druckschlauch (6) aufzunehmen, und

- wobei der optische Sensor (7; 223) in der Lage ist, die Veränderung der Intensität des Lichts der Lichtquelle (9; 221) in Reaktion auf die Veränderung des durch den Druckschlauch (6) auf die verformbare Seitenfläche der optischen Faser (80) aufgebrachten Drucks zu erkennen.

12. Druckwandler nach Anspruch 11, dadurch gekennzeichnet, daß die Lichtquelle (9) derart angeordnet ist, daß sie Licht am einen Ende der optischen Faser (80) einleitet, und daß der optische Sensor (7) derart angeordnet ist, daß er das durch die optische Faser (80) transmittierte und am anderen Ende der optischen Faser (80) ausgegebene Licht empfängt.

13. Druckwandler nach Anspruch 11, dadurch gekennzeichnet, daß die Lichtquelle (221) und der optische Sensor (223) beide derart angeordnet sind, daß sie an dem selben Ende der optischen Faser (80) Licht in die optische Faser (80) leiten bzw. das durch die optische Faser geleitete Licht empfangen, und daß der Druckwandler (8) ferner einen Reflektor (23) am anderen Ende der optischen Faser (80) aufweist.

14. Druckwandler nach Anspruch 13, dadurch gekennzeichnet, daß eine Lichtmeßeinheit (22), die eine Lichtquelle (221), einen optischen Sensor (223) und einen Lichtteiler (222) an einer ihrer Enden aufweist, und an ihrem anderen Ende mit einer Reflektionseinrichtung (23) zum Reflektieren des von der Lichtquelle (221) kommenden Lichts vorgesehen ist, daß der Druckschlauch (6) mit der optischen Faser (80) an einem Mittelbereich der optischen Faser (80) verbunden ist, daß das Licht von der Lichtquelle (221) durch den Lichtteiler (222) einfällt, und daß der optische Sensor (223) die Veränderung der Lichtintensität von Reflektionslicht, das von der Reflektionseinrichtung (23) reflektiert wird, erkennt, die aus der Veränderung eines Transmissionsfaktors der optischen Faser (80) resultiert, die sich in Reaktion auf die Veränderung des durch den Druckschlauch (6) auf die verformbare Seitenfläche der optischen Faser (80) aufgebrachten Drucks ergibt.

15. Druckwandler nach Anspruch 11, dadurch gekennzeichnet, daß die optische Faser (80) einen Kern (81) aus einem transparenten Elastomer mit einem hohen Refraktionsindex und einem Mantel (82) aus einem transparenten Elastomer mit einem niedrigen Refraktionsindex aufweist.

16. Druckwandler nach Anspruch 11, dadurch gekennzeichnet, daß die optische Faser (80) in einer Zelle (10) angeordnet ist und durch ein Füllmaterial (11) gehalten ist, das die optische Faser (80) an der Zelle (10) fixiert, und daß die Zelle (10) einen Flanschbereich (14) zum Verbinden des Druckschlauchs (6) mit dieser aufweist.

17. Druckwandler nach Anspruch 16, dadurch gekennzeichnet, daß die Zelle (10) ferner eine Empfindlichkeiteinstellschraube (13) zum Einstellen des Empfindlichkeitsbereichs der optischen Faser (80) aufweist.

18. Druckwandler nach Anspruch 15, dadurch gekennzeichnet, daß die optische Faser (80) ferner einen Hüllenbereich (83) aus einem opaken Elastomer aufweist.

19. Druckwandler nach Anspruch 11, dadurch gekennzeichnet, daß das transparente Elastomer aus der Gruppe gewählt ist, die aus Silikonelastomeren, Acrylatelastomeren, Fluorkarbonelastomeren, Vinylchloridelastomeren und Ethylen-Propylenelastomeren besteht.

20. Druckwandler nach Anspruch 15, dadurch gekennzeichnet, daß die Refraktionsindizes des Elastomers mit dem hohen Refraktionsindex und des Elastomers mit dem niedrigen Refraktionsindex einen Unterschied von wenigstens 0,02 aufweisen.

21. Verfahren zum Messen des Drucks eines Fluids mit den folgenden Schritten:

- Senden von Licht durch eine elastomere optische Faser (80),

- Verformen eines Teils der Seitenfläche der elastomeren optischen Faser (80) durch Beaufschlagen der Fläche mit druckbeaufschlagtem Fluid, und

- Erkennen der Intensität des durch die optische Faser (80) geleiteten Lichts durch einen optischen Sensor (7; 223), um so den Fluiddruck zu messen.

22. Verfahren nach Anspruch 21, gekennzeichnet durch die weiteren Schritte:

- Messen der Intensität des transmittierten Lichts, wenn die Seitenfläche der optischen Faser (80) mit einem ersten bekannten Druck beaufschlagt wird,

- Messen der Intensität des transmittierten Lichts, wenn die Seitenfläche der optischen Faser (80) mit einem zweiten bekannten Druck beaufschlagt wird,

- Berechnen des Verhältnisses zwischen den gemessenen Intensitäten und dem ersten und einem zweiten bekannten Druck, und

- Berechnen des Drucks eines Fluids durch Verwenden dieses Verhältnisses zum Umwandeln der Intensität des transmittierten Lichts, wenn die Seitenfläche der optischen Faser (80) mit dem Fluiddruck beaufschlagt ist.

23. Verfahren nach Anspruch 22, dadurch gekennzeichnet, daß die Berechnungsschritte unter Verwendung eines Mikroprozessors (45) durchgeführt werden.

**Revendications**

1. Un système transducteur de pression sanguine, comprenant:

- un cathéter (1a),
- Un tube de pression (6) raccordé à une des extrémités dudit cathéter (1a), ledit tube de pression (6) et ledit cathéter étant en communication fluidique, et
- un transducteur de pression (8) raccordé à l'autre extrémité dudit tube de pression (6), dans lequel ledit transducteur de pression (8) comporte une fibre optique (80) formée en un élastomère transparent et ayant une surface latérale déformable, dans lequel ledit transducteur de pression (8) comportre une source lumineuse (9; 221) et un détecteur optique (7;223), dans lequel ledit tube de pression (6) est raccordé à ladite fibre optique (80) dans un emplacement intermédiaire de ladite fibre optique, et dans lequel ledit détecteur optique (7; 223) est capable de détecter le changement d'intensité de la lumière de ladite source lumineuse (9; 221) en réponse au changement de pression exercé par l'intermédiaire dudit tube de pression (6) sur la surface latérale déformable de ladite fibre optique.

2. Un transducteur de pression selon la revendication 1, caractérisé en ce que ladite source lumineuse (9) est agencée de façon à introduire la lumière à une extrémité de ladite fibre optique (80) et en ce que ledit détecteur optique (7) est agencé de façon à recevoir la lumière transmise par ladite fibre optique (80) et sortant par l'autre extrémité de ladite fibre optique (80).

3. Un transducteur de pression selon la revendication 1, caractérisé en ce que ladite source lumineuse (221) et ledit détecteur optique (223) sont tous deux agencés de façon à introduire de la lumière dans ladite fibre optique et, respectivement, à recevoir la lumière transmise par ladite fibre optique (80) à partir de la même extrémité de ladite fibre optique (80) et en ce que ledit transducteur de pression (8) comporte, en outre, un réflecteur (23) à l'autre extrémité de ladite fibre optique (80).

4. Un transducteur de pression selon la revendication 3, caractérisé en ce que ledit transducteur de pression (8) est équipé d'un dispositif de mesure de la lumière (22) qui comporte une source lumineuse (221), un détecteur optique (223) et un diviseur de lumière (222) à une de ses extrémités et des moyens de réflexion à son autre extrémité pour réfléchir une lumière provenant de ladite source lumineuse (221), ledit tube de pression (6) est raccordé à ladite fibre optique (80) dans une partie intermédiaire de ladite fibre optique (80), ladite lumière est amenée à être incidente en provenance de ladite source lumineuse (221) à travers ledit diviseur de lumière (222), et ledit détecteur optique (223) détecte le changement de l'intensité lumineuse de la lumière de réflexion réfléchie par lesdits moyens de réflexion (23) qui résulte du changement d'un facteur de transmission de ladite fibre optique (80) en réponse au changement de la pression exercée par l'intermédiaire dudit tube de pression (6) sur la surface latérale déformable de ladite fibre optique (80).

5. Un transducteur de pression selon la revendication 1, caractérisé en ce que ladite fibre optique comporte une partie d'âme (81) formée en un élastomère transparent ayant un indice de réfraction élevé et une partie d'enveloppe (82) formée en un élastomère transparent ayant un faible indice de réfraction.

6. Un transducteur de pression selon la revendication 1, caractérisé en ce que ladite fibre optique (80) est disposée dans une cellule (10) et est maintenue en place par une matière de garniture (11) qui solidarise la fibre optique (80) de la cellule, et en ce que ladite cellule (10) comporte une partie de collerette (14) pour permettre le raccordement dudit tube de pression (6) à ladite cellule.

7. Un transducteur de pression selon la revendication 6, caractérisé en ce que ladite cellule (10) comporte, en outre, une vis de réglage de sensibilité (13) pour ajuster la variance de sensibilité de ladite fibre optique (80).

8. Un transducteur de pression selon la revendication 5, caractérisé en ce que ladite fibre optique (80) comporte, en outre, une partie de gaine (83) formée en un élastomère opaque.

9. Un transducteur de pression selon la revendication 1, caractérisé en ce que ledit élastomère transparent est choisi dans le groupe composé des élastomères de silicone, des élastomères d'acrylate, des élastomères de fluorocarbone, des élastomères de chlorure de vinyle et des élastomères d'éthylène propylène.

10. Un transducteur de pression selon la revendication 5, caractérisé en ce que les indices de réfraction dudit élastomère à indice de réfraction élevé et dudit élastomère à faible indice de réfraction présentent une différence d'au moins 0,02.

11. Un transducteur de pression comprenant:

- une fibre optique (80) formée en un élastomère transparent et ayant une surface latérale déformable,
- une source lumineuse (9; 221), et
- un détecteur optique (7; 223),
- dans lequel ladite fibre optique (80) est agencée pour recevoir un tube de pression (6) en un emplacement intermédiaire de ladite fibre optique (80), et
- dans lequel ledit détecteur (7; 223) est capable de détecter le changement de l'intensité de la lumière de ladite source lumineuse (9; 221) en réponse au changement de la pression exercée par l'intermédiaire dudit tube de pression (6) sur la surface latérale de ladite fibre optique (80).

12. Un transducteur de pression selon la revendication 11, caractérisé en ce que ladite source lumineuse (9) est agencée de façon à introduire la lumière à une extrémité de ladite fibre optique (80) et en ce que ledit détecteur optique (7) est agencé de façon à recevoir la lumière transmise par ladite fibre optique (80) et sortant par l'autre extrémité de ladite fibre optique (80).

13. Un transducteur de pression selon la revendication 11, caractérisé en ce que ladite source lumineuse (221) et ledit détecteur optique (223) sont tous deux agencés de façon à introduire de la lumière dans ladite fibre optique et, respectivement, à recevoir la lumière transmise par ladite fibre optique (80) à partir de la même extrémité de ladite fibre optique (80) et en ce que ledit transducteur de pression (8) comporte, en outre, un réflecteur (23) à l'autre extrémité de ladite fibre optique (80).

14. Un transducteur de pression selon la revendication 13, caractérisé en ce qu'il est prévu un dispositif de mesure de la lumière (22) qui comporte une source lumineuse (221), un détecteur optique (223) et un diviseur de lumière (222) à une de ses extrémités et des moyens de réflexion à son autre extrémité pour réfléchir une lumière provenant de ladite source lumineuse (221), ledit tube de pression (6) est raccordé à ladite fibre optique (80) dans une partie intermédiaire de ladite fibre optique (80), ladite lumière est amenée à être incidente en provenance de ladite source lumineuse (221) à travers ledit diviseur de lumière (222), et ledit détecteur optique (223) détecte le changement de l'intensité lumineuse de la lumière de réflexion réfléchie par lesdits moyens de réflexion (23) qui résulte du changement d'un facteur de transmission de ladite fibre optique (80) en réponse au changement de la pression exercée par l'intermédiaire dudit tube de pression (6) sur la surface latérale déformable de ladite fibre optique (80).

15. Un transducteur de pression selon la revendication 11, caractérisé en ce que ladite fibre optique comporte une partie d'âme (81) formée en un élastomère transparent ayant un indice de réfraction élevé et une partie d'enveloppe (82) formée en un élastomère transparent ayant un faible indice de réfraction.

16. Un transducteur de pression selon la revendication 11, caractérisé en ce que ladite fibre optique (80) est disposée dans une cellule (10) et est maintenue en place par une matière de garniture (11) qui solidarise la fibre optique (80) de la cellule, et en ce que ladite cellule (10) comporte une partie de collerette (14) pour permettre le raccordement dudit tube de pression (6) à ladite cellule.

17. Un transducteur de pression selon la revendication 16, caractérisé en ce que ladite cellule (10) comporte, en outre, une vis de réglage de sensibilité (13) pour ajuster la variance de sensibilité de ladite fibre optique (80).

**18.** Un transducteur de pression selon la revendication 15, caractérisé en ce que ladite fibre optique (80) comporte, en outre, une partie de gaine (83) formée en un élastomère opaque.

**19.** Un transducteur de pression selon la revendication 11, caractérisé en ce que ledit élastomère transparent est choisi dans le groupe composé des élastomères de silicone, des élastomères d'acrylate, des élastomères de fluorocarbone, des élastomères de chlorure de vinyle et des élastomères d'éthylène propylène.

**20.** Un transducteur de pression selon la revendication 15, caractérisé en ce que les indices de réfraction dudit élastomère à indice de réfraction élevé et dudit élastomère à faible indice de réfraction présentent une différence d'au moins 0,02.

**21.** Un procédé pour mesurer la pression d'un fluide comportant les étapes qui consistent:

- à transmettre une lumière à travers une fibre optique (80) en matière élastomère,
- à déformer une partie de la surface latérale de ladite fibre optique (80) en matière élastomère en appliquant un fluide sous pression sur ladite surface, et
- à détecter l'intensité de la lumière transmise par ladite fibre optique (80) au moyen d'un détecteur optique (7; 223) de façon à mesure la pression du fluide.

**22.** Un procédé selon la revendication 21, caractérisé par les étapes supplémentaires suivantes qui consistent:

- à mesurer l'intensité de ladite lumière transmise lorsque la surface latérale de ladite fibre optique (80) est soumise à une première pression connue,
- à mesurer l'intensité de ladite lumière transmise lorsque la surface latérale de ladite fibre optique (80) est soumise à une seconde pression connue,
- à calculer le rapport entre lesdites intensités mesurées et lesdites première et seconde pressions connues, et
- à calculer la pression d'un fluide en utilisant ledit rapport pour convertir l'intensité de la lumière transmise lorsque ladite surface latérale de ladite fibre optique (80) est soumise à la pression dudit fluide.

**23.** Un procédé selon la revendication 22, caractérisé en ce que lesdites étapes de calcul sont effectuées en utilisant un microprocesseur (45).

**Fig. 1**

**Fig. 2**

**Fig. 3**

**Fig. 4**

1sec

100
mV

**Fig. 5A**

200
mV

**Fig. 5B**

(mV)

0

-5

-10

-15

I

II

III

0          50          100         150

(mmHg)

**Fig. 6**

## Fig. 8

## Fig. 9

**Fig. 7**

**Fig. 10**

**Fig. 11**